# FASCICULE DE BREVET EUROPEEN

(11) **EP 0 593 334 B1**
(45) Date de publication et mention de la délivrance du brevet: **28.05.1997**
(21) Numéro de dépôt: 93402435.7
(22) Date de dépôt: 05.10.1993
(51) Int. Cl.: C07C 263/10, C07C 265/14

(54) **Procédé de préparation de composés du type isocyanates aromatiques en phase gazeuse**
Verfahren zur Herstellung von aromatischen Isocyanat-Verbindungen in gasförmiger Phase
Process for the preparation of aromatic isocyanate compounds in gazeous phase

(30) Priorité: 16.10.1992 FR 9212436
(43) Date de publication de la demande: 20.04.1994
(73) Titulaire: RHONE-POULENC CHIMIE, 92408 Courbevoie Cédex (FR)
(72) Inventeur: Joulak, Faouzi,, F-78150 Le Chesnay (FR); Revelant, Denis, F-69740 Genas (FR); Vacus, Pascale, F-69100 Villeurbanne (FR)
(74) Mandataire: Dubruc, Philippe

(56) Documents cités:
- EP-A- 0 289 840
- GB-A- 656 726
- GB-A- 1 165 831
- US-A- 2 480 089

## Description

La présente invention concerne un procédé de préparation de composés aromatiques substitués par au moins deux groupements isocyanates. Elle concerne plus particulièrement un procédé de préparation de toluène diisocyanate, ses isomères seuls ou en mélange.

La préparation de composés aromatiques substitués par un ou plusieurs groupements isocyanates par réaction d'amines avec du phosgène en phase gazeuse est connue depuis longtemps, bien qu'elle n'ait eu de véritable essor que pour la transformation d'amines monofonctionnelles.

Ainsi, dans la demande de brevet GB 656 726, au nom de Monsanto, on a décrit la phosgénation, en phase gazeuse, de monoamines aliphatiques, comme la méthylamine ou l'éthylamine, ou de monoamines aromatiques comme l'aniline. Cependant, la réaction d'amines polyfonctionnelles avec le phosgène n'est pas abordée.

La demande de brevet GB 1 165 831, au nom d'ICI, décrit la réaction en phase gazeuse d'amines mono- ou polyfonctionnelles aromatiques ou aliphatiques avec le phosgène, en vue de préparer les isocyanates correspondants, la réaction ayant lieu dans un réacteur cylindrique muni d'un mobile d'agitation mécanique. Un tel procédé présente des inconvénients liés à l'utilisation de pièces tournantes de l'agitateur mécanique, tels que notamment des problèmes d'étanchéité au niveau de l'axe de rotation, des problèmes d'encrassement et donc de blocage du mobile d'agitation dûs à des sous-produits de réactions collants.

La présente invention a donc pour objet un procédé ne présentant pas les inconvénients précédemment indiqués et concernant la préparation de composés aromatiques subsitués par au moins deux groupements isocyanates. Le procédé selon l'invention est caractérisé en ce que l'on met en contact, en phase gazeuse, au moins un composé (A) comprenant au moins deux fonctions amine primaire et au moins un motif aromatique, avec du phosgène, et en ce que le réacteur, dans lequel la mise en contact de ces réactifs est effectuée, est dépourvu de mobile d'agitation.

Le procédé de préparation selon l'invention permet donc d'obtenir les isocyanates correspondant à la transformation des amines précitées sans qu'il soit nécessaire d'effectuer la réaction dans un réacteur muni de mobile d'agitation. Ainsi, les réacteurs comprenant des mobiles d'agitation, mécaniques comme ceux décrits notamment dans la demande de brevet GB 1 165 831, ne sont plus nécessaires.

Le procédé de préparation selon l'invention va maintenant être décrit plus en détails.

Ainsi qu'il a été dit plus haut, le procédé selon l'invention comprend la mise en contact, en phase gazeuse, d'au moins un composé (A) présentant au moins deux fonctions amine primaire et au moins un motif aromatique, avec du phosgène.

Selon un mode particulier de réalisation de l'invention, on met en oeuvre le procédé en utilisant au moins un composé (A) comprenant au moins deux fonctions amine primaire et au moins un motif aromatique en C₆-C₁₄, de préférence en C₆-C₁₀, substitué ou non par un ou plusieurs radicaux hydrocarbonés, saturés ou non, linéaires, cycliques ou ramifiés en C₁-C₁₀.

Plus précisément, les radicaux hydrocarbonés précités, substituant éventuellement lesdits motifs aromatiques, peuvent être choisis parmi les radicaux alkyle, aryle, alkyle-aryle et aryle-alkyle en C₁-C₁₀, de préférence en C₁-C₆.

De préférence, le composé (A) correspond au produit de formule :

H₂N-R-NH₂ (1),

formule (1) dans laquelle R représente le motif aromatique, substitué ou non, décrit ci-dessus.

Selon un mode plus particulier de réalisation de l'invention, on utilise au moins un composé (A) dans lequel le motif R précité est éventuellement substitué par un ou plusieurs radicaux alkyle en C₁-C₁₀, de préférence en C₁-C₆.

Comme motif R, on peut notamment citer les noyaux benzénique et naphtalénique substitués ou non par un ou plusieurs radicaux méthyle, éthyle, propyle, butyle, pentyle, hexyle et/ou leurs isomères.

De façon préférée, le procédé est mis en oeuvre en utilisant au moins un composé (A) choisi parmi la toluène diamine, la xylylène diamine, la phénylène diamine ; ces composés pouvant être utilisés seuls ou en mélange, avec ou sans leurs isomères

Le composé (A), de façon encore plus préférée, est la toluène diamine.

D'une manière générale, le procédé selon l'invention est réalisé avec un excès de phosgène variant entre 0 et 300 % en mole, rapporté au nombre de fonctions amine présentes dans le composé (A). De préférence, on utilise un excès de phosgène variant entre 10 et 300 % et plus particulièrement variant entre 10 et 200 % en mole, rapporté en nombre de fonctions amine du composé (A).
Il est à noter que des quantités plus importantes n'apportent rien de plus à la réaction, notamment en terme de rendement.

Les réactifs mis en contact dans la réaction selon l'invention, c'est-à-dire au moins un composé (A) et le phosgène, peuvent être engagés seuls ou en présence d'un gaz vecteur diluant. Par gaz vecteur diluant, on entend tout gaz diluant et inerte vis-à-vis des réactifs et des produits de la réaction. Outre les gaz inertes, comme notamment l'azote, on peut également utiliser la vapeur d'un solvant servant à diluer le composé (A) et/ou le phosgène. Le solvant précité peut notamment être choisi parmi le benzène, le xylène, l'orthodichlorobenzène, le monochlorobenzène.

Dans le cas où le composé (A) est solubilisé dans un solvant, celui-ci se trouve présent, préalablement à la mise en contact avec le phosgène, à une concentration massique comprise entre 3 et 30 % dans ledit solvant. De préférence, cette concentration est comprise entre 10 et 20 %.

Ainsi qu'il a été dit auparavant, la réaction selon l'invention est réalisée dans un réacteur dépourvu de mobile d'agitation. Par mobile d'agitation, on entend classiquement des moyens d'agitation mécaniques mobiles.

La réaction selon l'invention peut être réalisée dans tout type de réacteur dont le matériau est compatible avec les conditions opératoires. Ainsi, la réaction peut être réalisée notamment dans un réacteur dont le matériau est du verre ou de l'acier, allié ou émaillé.

Quant à la forme du réacteur, on effectue la réaction selon l'invention dans un réacteur tubulaire présentant un étranglement des parois.

L'introduction des réactifs peut être réalisée de différentes manières. On les introduit, de préférence, par injection au moyen d'une buse. Cette buse peut être, par exemple, composée de deux tubes concentriques insérés l'un dans l'autre formant une partie centrale et une partie annulaire. Le composé (A) et le phosgène, éventuellement dilués dans un gaz vecteur, peuvent ainsi être indifféremment introduits par la partie centrale ou la partie annulaire.

Selon un mode de mise en oeuvre particulier et préféré de l'invention, les réactifs sont introduits dans des conditions telles que l'on ait un régime turbulent au niveau de la zone de contact desdits réactifs. Toutefois, on peut noter qu'une introduction des réactifs en régime laminaire n'est pas exclue.

Plus particulièrement, le nombre de Reynolds du mélange des réactifs au niveau de la zone de contact est d'au moins 3000, de préférence d'au moins 5000 et plus particulièrement d'au moins 8000.

A titre d'exemple, une telle valeur du nombre de Reynolds peut se traduire notamment par une vitesse du mélange gazeux des réactifs introduits comprise entre 3 et 15 m/s pour un diamètre de tube compris entre 2 et 6 mm. Ces faibles vitesses des gaz présentent l'avantage de limiter les phénomènes d'érosion du matériau du réacteur.

Outre la vitesse du mélange gazeux des réactifs, ce régime turbulent peut être de même obtenu avec une géométrie particulière du réacteur comme l'étranglement des parois du réacteur ou encore avec tout arrangement d'obstacles internes statiques, comme les chicanes par exemple. On peut aussi envisager de combiner la vitesse du mélange gazeux des réactifs et la géométrie du réacteur, comprenant éventuellement des obstacles internes, ou, tout autre moyen connu de l'homme du métier.

D'une manière simple et avantageuse, il n'est pas nécessaire de maintenir le mélange dans un régime turbulent, une fois réalisée la mise en contact des réactifs. Ainsi, un écoulement voisin du type piston convient particulièrement bien, le taux de conversion augmentant le long de l'axe du réacteur. De cette manière, la vitesse du mélange, après la zone de contact et jusqu'à la sortie du réacteur, peut varier de 15 m/s à une vitesse aussi faible que 0,5 m/s pour les diamètres précités.

Le temps de séjour des réactifs dans le réacteur est, de préférence, compris entre 1 et 15 secondes. Selon un mode particulier de réalisation de l'invention, le temps de séjour des réactifs dans le réacteur est compris entre 3 et 6 secondes.

Il a été trouvé d'une façon surprenante que des temps de séjour de cet ordre de grandeur ne diminuent pas les rendements de la réaction de phosgénation, malgré le caractère thermosensible des réactifs et des produits obtenus.

La température à laquelle la réaction de phosgénation selon l'invention est effectuée, varie habituellement entre 250 et 500°C. Plus particulièrement ladite température de réaction est comprise entre 300 et 400°C. Par température de réaction, on entend la température régnant dans le réacteur.

Selon un mode de réalisation particulier de l'invention, les réactifs sont préchauffés avant leur mise en contact dans le réacteur. Habituellement, la température de préchauffage des réactifs est du même ordre de grandeur que celle requise pour effectuer la phosgénation.

Dans le cas où le composé (A) est utilisé en présence d'un solvant, on effectue en général au préalable la solubilisation, en phase liquide, dudit réactif dans son solvant. Le mélange résultant est ensuite vaporisé à la température requise pour la réaction, par tout moyen connu de l'homme du métier.

Le procédé selon l'invention peut être réalisé indifféremment sous pression, à pression réduite ou à pression atmosphérique. A titre d'exemple, la pression régnant dans le réacteur peut varier entre 0,5 et 1,5 bar absolus. De préférence, on procède à une pression voisine de la pression atmosphérique.

Une fois la réaction de phosgénation réalisée, les produits obtenus ainsi que les réactifs n'ayant pas réagi sont séparés selon toute méthode connue de l'homme du métier.

On pourra par exemple séparer l'isocyanate produit par condensation sélective de celui-ci dans un solvant adéquat.

Pour des questions de commodité, ledit solvant est de préférence choisi de telle sorte que sa température d'ébullition est supérieure à celle de la décomposition du chlorure de carbamyle correspondant à l'isocyanate formé. Choisir un solvant répondant à ce critère évite une étape ultérieure de décomposition dudit chlorure de carbamyle.

Par ailleurs, ce même solvant doit pouvoir, de préférence, se condenser à une température pour laquelle les produits, comme notamment le phosgène restant et l'acide chlorhydrique formé, restent à l'état gazeux.

L'isocyanate récupéré est alors purifié, notamment par distillation.

En ce qui concerne les produits comme le phosgène ou l'acide chlorhydrique, ceux-ci peuvent être détruits par traitement avec une base telle que la soude. Ils peuvent de même être séparés par tout moyen connu en soi, pour être ensuite réutilisés.

Par exemple, le phosgène peut être séparé de l'acide chlorhydrique par distillation, par adsorption dans un solvant à basse température, puis recyclé dans le procédé de phosgénation.

Des exemples concrets mais non limitatifs de réalisation de l'invention vont maintenant être présentés.

### EXEMPLE 1

Dans un réacteur tubulaire en inox de 250 cm³ (longueur : 60 cm et diamètre : 2,3 cm) chauffé électriquement à 320°C, on introduit:
- par la partie centrale de l'injecteur, de la 2-4 toluène diamine préchauffée à 320°C et diluée dans le l'o-dichlorobenzène avec des débits respectifs de 102,5 g/h et 922,5 g/h;
- par la partie annulaire de l'injecteur, du phosgène gazeux préchauffé à 320°C avec un débit de 515,6 g/h (soit un excès d'environ 200 % molaire par rapport au nombre de fonctions amine).

Le nombre de Reynolds, après injection, du mélange gazeux est de 9000 au niveau de la zone de contact (correspondant à une vitesse des gaz de 12 m/s) et de 2600 à la sortie du réacteur (correspondant à une vitesse des gaz de 0,5 m/s).

Le temps de séjour dans le réacteur est de 1,8 secondes.

La pression dans le réacteur est de 1,3 bar.a. et la température de 320°C.

Le mélange gazeux sortant du réacteur est introduit dans une colonne remplie avec de l'o-dichlorobenzène à 180°C à reflux. Le toluène di-isocyanate (eb = 250°C sous 1 bar.a.) est condensé et récupéré en pied de colonne.

Les incondensables (acide chlorhydrique, phosgène) sont véhiculés vers une colonne d'abattage à la soude.

Le rendement en toluène di-isocyanate par rapport à la toluène diamine introduite dans le réacteur est de 92% avec un taux de transformation de 100%.

Le taux de résidus [résidus/(résidus + toluène di-isocyanate)] mesuré par microdistillation est de 7,5%.

### EXEMPLE 2

Dans un réacteur identique à celui de l'exemple 1 et chauffé à 320°C, on introduit:
- par la partie centrale de l'injecteur, 90 g/h de m-phénylène diamine et 810 g/h d'o-dichlorobenzène vaporisés à 320°C;
- par la partie annulaire de l'injecteur, 515,6 g/h de phogène gazeux préchauffé à 320°C (soit un excès d'environ 200 % molaire par rapport au nombre de fonctions amine).

Le temps de séjour dans le réacteur est de 2 secondes.

Les autres conditions opératoires sont par ailleurs identiques à celles de l'exemple 1.

Le rendement en m-phénylène diisocyanate est de 93% par rapport à la m-phénylène diamine introduite avec un taux de résidus de 5,8%.

## Revendications

1. Procédé de préparation de composés aromatiques substitués par au moins deux groupements isocyanates, caractérisé en ce que l'on met en contact, en phase gazeuse, au moins un composé (A) comprenant au moins deux fonctions amine primaire et au moins un motif aromatique, avec du phosgène, et en ce que le réacteur dans lequel on effectue ladite mise en contact, est un réacteur tubulaire, dépourvu de mobile d'agitation et présentant un étranglement des parois.

2. Procédé selon la revendication 1, caractérisé en ce que l'on utilise au moins un composé (A) comprenant au moins deux fonctions amine primaire et au moins un motif aromatique en C₆-C₁₄, de préférence en C₆-C₁₀, substitué ou non par un ou plusieurs radicaux hydrocarbonés, saturés ou non, linéaires, cycliques ou ramifiés en C₁-C₁₀, de préférence en C₁-C₆.

3. Procédé selon la revendication précédente, caractérisé en ce que l'on utilise au moins un composé (A) dans lequel le motif aromatique précité peut être éventuellement substitué par un ou plusieurs radicaux hydrocarbonés choisis parmi les radicaux alkyles, aryles, alkyle-aryles et aryle-alkyles en C₁-C₁₀, de préférence en C₁-C₆.

4. Procédé selon l'une quelconque des revendications précédentes, caractérisé en ce que l'on utilise au moins un composé (A) correspond au produit de formule :
H₂N-R-NH₂ (1),
formule (1) dans laquelle R représente un motif aromatique tel que décrit dans l'une des revendications 2 ou 3.

5. Procédé selon la revendication précédente, caractérisé en ce que l'on utilise au moins un composé (A) de formule (1) dans laquelle R représente un motif aromatique en C₆-C₁₄, de préférence en C₆-C₁₀, substitué ou non par un ou plusieurs radicaux alkyles en C₁-C₁₀, et de préférence en C₁-C₆.

6. Procédé selon la revendication précédente, caractérisé en ce que l'on utilise au moins un composé (A) choisi parmi la toluène diamine, la xylylène diamine et la phénylène diamine seuls ou en mélange, avec ou sans leurs isomères.

7. Procédé selon l'une quelconque des revendications précédentes, caractérisé en ce que l'on utilise un excès de phosgène variant entre 0 et 300 % en mole, rapporté au nombre de fonctions amine présentes dans le composé (A).

8. Procédé selon l'une quelconque des revendications précédentes, caractérisé en ce que l'on utilise un excès en phosgène variant de préférence entre 10 à 300% et plus particulièrement variant entre 10 et 200 % en mole, rapporté au nombre de moles de fonctions amines présentes dans le composé (A).

9. Procédé selon l'une quelconque des revendications précédentes, caractérisé en ce que l'on utilise au moins un composé (A) et/ou le phosgène, seuls ou en présence d'un gaz vecteur diluant.

10. Procédé selon la revendication précédente, caractérisé en ce que l'on utilise le ou les composés (A) avec une concentration massique comprise entre 3 et 30%, de préférence entre 10 et 20%, dans ledit solvant.

11. Procédé selon l'une quelconque des revendications précédentes, caractérisé en ce que l'on introduit les réactifs, éventuellement dilués, dans des conditions telles que l'on ait un régime turbulent au niveau de la zone de contact desdits réactifs.

12. Procédé selon la revendication précédente, caractérisé en ce que l'on introduit les réactifs précités dans des conditions telles que le nombre de Reynolds du mélange des réactifs au niveau de la zone de contact, est d'au moins 3000, de préférence d'au moins 5000, et plus particulièrement d'au moins 8000.

13. Procédé selon la revendication précédente, caractérisé en ce que l'on introduit les réactifs dans des conditions telles que la vitesse du mélange gazeux des réactifs introduits est comprise entre 3 et 15 m/s pour un diamètre de tube du réacteur compris entre 2 et 6 mm.

14. Procédé selon l'une quelconque des revendications 1 à 11 caractérisé en ce que l'on introduit les réactifs, éventuellement dilués, dans des conditions telles que l'on ait un régime laminaire au niveau de la zone de contact desdits réactifs.

15. Procédé selon l'une quelconque des revendications précédentes, caractérisé en ce que l'on confère au mélange des gaz obtenu après la mise en contact des réactifs, un écoulement voisin du type piston.

16. Procédé selon l'une quelconque des revendications précédentes, caractérisé en ce que le temps de séjour des réactifs dans le réacteur est compris entre 1 et 15 secondes, de préférence compris entre 3 et 6 secondes.

17. Procédé selon l'une quelconque des revendications précédentes, caractérisé en ce que l'on effectue la mise en contact des réactifs à une température comprise entre 250 et 500°C, plus particulièrement à une température comprise entre 300 et 400°C.

18. Procédé selon l'une quelconque des revendications précédentes, caractérisé en ce que l'on met en oeuvre ledit procédé à une pression voisine de la pression atmosphérique.

19. Procédé selon l'une quelconque des revendications précédentes, caractérisé en ce que l'on sépare le composé aromatique substitué par au moins deux groupements isocyanates, obtenu par le procédé selon l'une quelconque des revendications précédentes, par condensation sélective de celui-ci dans un solvant dont la température d'ébullition est supérieure à la température de décomposition du chlorure de carbamyle correspondant au composé aromatique précité.

## Claims

1. A process for the preparation of aromatic compounds which are substituted by at least two isocyanate groups, characterised in that at least one compound (A) comprising at least two primary amine functions and at least one aromatic unit is brought into contact in the gaseous phase with phosgene, and that the reactor in which said contacting operation is effected is a tubular reactor which does not have movable agitation means and which has a constriction in respect of the walls thereof.

2. A process according to claim 1 characterised by using at least one compound (A) comprising at least two primary amine functions and at least one aromatic C₆-C₁₄ and preferably C₆-C₁₀ unit which may or may not be substituted by one or more saturated or unsaturated, straight-chain, cyclic or branched C₁-C₁₀ and preferably C₁-C₆ hydrocarbon radicals.

3. A process according to the preceding claim characterised by using at least one compound (A) in which said aromatic unit may optionally be substituted by one or more hydrocarbon radicals selected from C₁-C₁₀ and preferably C₁-C₆ alkyl, aryl, alkyl-aryl and aryl-alkyl radicals.

4. A process according to any one of the preceding claims characterised by using at least one compound (A) corresponding to the product of the following formula:
H₂N-R-NH₂ (1),
in which formula (1) R represents an aromatic unit as described in one of claims 2 and 3.

5. A process according to the preceding claim characterised by using at least one compound (A) of formula (1) in which R represents a C₆-C₁₄ and preferably C₆-C₁₀ aromatic unit which may or may not be substituted by one or more C₁-C₁₀ and preferably C₁-C₆ alkyl radicals.

6. A process according to the preceding claim characterised by using at least one compound (A) selected from toluene diamine, xylylene diamine and phenylene diamine alone or in the form of a mixture, with or without their isomers.

7. A process according to any one of the preceding claims characterised by using an excess of phosgene varying between 0 and 300 molar % with respect to the number of amine functions present in the compound (A).

8. A process according to any one of the preceding claims characterised by using an excess of phosgene varying preferably between 10 and 300 molar % and are particularly varying between 10 and 200 molar %, with respect to the number of ales of amine functions present in the compound (A).

9. A process according to any one of the preceding claims characterised by using at least one compound (A) and/or phosgene, alone or in the presence of a diluent carrier gas.

10. A process according to the preceding claim characterised by using the compound or compounds (A) with a concentration by mass of between 3 and 30% and preferably between 10 and 20% in said solvent.

11. A process according to any one of the preceding claims characterised in that the reactants, optionally diluted, are introduced under conditions such as to provide a turbulent system at the location of the contact zone of said reactants.

12. A process according to the preceding claim characterised in that said reactants are introduced under conditions such that the Reynolds number of the mixture of reactants at the location of the contact zone is at least 3000 and preferably at least 5000 and more particularly at least 8000.

13. A process according to the preceding claim characterised in that the reactants are introduced under conditions such that the speed of the gaseous mixture of the reactants introduced is between 3 and 15 m/s for a diameter of the tube of the reactor of between 2 and 6 mm.

14. A process according to any one of claims 1 to 11 characterised in that the reactants, optionally diluted, are introduced under conditions such that there is a laminar system at the location of the contact zone of said reactants.

15. A process according to any one of the preceding claims characterised in that a flow similar to the piston type is imparted to the mixture of gases which is obtained after the reactants are brought into contact.

16. A process according to any one of the preceding claims characterised in that the residence time of the reactants in the reactor is between 1 and 15 seconds and preferably between 3 and 6 seconds.

17. A process according to any one of the preceding claims characterised in that the operation of bringing the reactants into contact is effected at a temperature of between 250 and 500°C and more particularly at a temperature of between 300 and 400°C.

18. A process according to any one of the preceding claims characterised in that said process is effected at a pressure close to atmospheric pressure.

19. A process according to any one of the preceding claims characterised in that the aromatic compound which is substituted by at least two isocyanate groups and which is obtained by the process according to any one of the preceding claims is separated by selective condensation thereof in a solvent whose boiling temperature is higher than the decomposition temperature of carbamyl chloride corresponding to said aromatic compound.

## Patentansprüche

1. Verfahren zur Herstellung von aromatischen Verbindungen, die durch mindestens zwei Isocyanatgruppen substituiert sind, dadurch gekennzeichnet, daß man in der Gasphase mindestens eine Verbindung (A), welche mindestens zwei primäre Aminfunktionen und mindestens eine aromatische Grundeinheit hat, mit Phosgen in Kontakt bringt, und daß der Reaktor, worin dieses Kontaktieren durchgeführt wird, ein Rohrrektor ist, ohne bewegliches Rühren und eine Einengung der Wände aufweisend.

2. Verfahren gemäß Anspruch 1, dadurch gekennzeichnet, daß man mindestens eine Verbindung (A) verwendet, die mindestens zwei primäre Aminfunktionen und mindestens eine aromatische C₆-C₁₄-, vorzugsweise C₆-C₁₀-aromatische Grundeinheit, substituiert oder nichtsubstituiert durch ein oder mehrere gesättigte oder nichtgesättigte, lineare, cyclische oder verzweigte C₁-C₁₀-, vorzugsweise C₁-C₆-Kohlenwasserstoffreste substituiert ist.

3. Verfahren gemäß dem vorhergehenden Anspruch, dadurch gekennzeichnet, daß man mindestens eine Verbindung (A) verwendet, worin die vorerwähnte aromatische Grundeinheit gegebenenfalls substituiert sein kann durch ein oder mehrere Kohlenwasserstoffreste, ausgewählt unter den C₁-C₁₀-, vorzugsweise C₁-C₆-Alkyl-, -Aryl-, -Alkylaryl- und Arylalkylresten.

4. Verfahren gemäß einem der vorhergehenden Ansprüche, dadurch gekennzeichnet, daß man mindestens eine Verbindung (A) verwendet, die dem Produkt der Formel
H₂N-R-NH₂ (1),
entspricht, wobei in der Formel (1) R eine aromatische Grundeinheit darstellt, wie sie in einem der Ansprüche 2 oder 3 beschrieben ist.

5. Verfahren gemäß dem vorhergehenden Anspruch, dadurch gekennzeichnet, daß man mindestens eine Verbindung (A) entsprechend der Formel (1) verwendet, worin R eine aromatische C₆-C₁₄-, vorzugsweise C₆-C₁₀-aromatische Grundeinheit, substituiert oder nichtsubstituiert durch einen oder mehrere C₁-C₁₀-, vorzugsweise C₁-C₆-Alkylreste darstellt.

6. Verfahren gemäß dem vorhergehenden Anspruch, dadurch gekennzeichnet, daß man mindestens eine Verbindung (A) verwendet, ausgewählt unter Toluoldiamin, Xylylendiamin und Phenylendiamin, alleine oder im Gemisch mit oder ohne ihre Isomeren.

7. Verfahren gemäß einem der vorhergehenden Ansprüche, dadurch gekennzeichnet, daß man einen Überschuß an Phosgen verwendet, variierend zwischen 0 und 300 Mol-%, bezogen auf die in der Verbindung (A) vorliegenden Aminfunktionen.

8. Verfahren gemäß einem der vorhergehenden Ansprüche, dadurch gekennzeichnet, daß man einen Überschuß an Phosgen verwendet, variierend vorzugsweise zwischen 10 bis 300 Mol-%, und insbesondere variierend zwischen 10 und 200 Mol-%, bezogen auf die Zahl der Mole der in der Verbindung (A) vorhandenen Aminfunktionen.

9. Verfahren gemäß einem der vorhergehenden Ansprüche, dadurch gekennzeichnet, daß man mindestens eine Verbindung (A) und/oder Phosgen verwendet, alleine oder in Anwesenheit eines Gasverdünnungsvektors.

10. Verfahren gemäß dem vorhergehenden Anspruch, dadurch gekennzeichnet, daß man die Verbindung(en) (A) in einer Massenkonzentration zwischen 3 und 30 %, vorzugsweise zwischen 10 und 20 %, in dem genannten Lösungsmittel verwendet.

11. Verfahren gemäß einem der vorhergehenden Ansprüche, dadurch gekennzeichnet, daß man die gegebenenfalls verdünnten Reagentien unter solchen Bedingungen einführt, daß man auf dem Niveau der Kontaktzone dieser Reagentien einen turbulenten bzw. wirbelnden Betriebszustand hat.

12. Verfahren gemäß dem vorhergehenden Anspruch, dadurch gekennzeichnet, daß man die vorerwähnten Reagentien unter solchen Bedingungen einführt, daß die Reynolds-Zahl des Gemisches der Reagentien auf dem Niveau der Kontaktzone mindestens 3000, vorzugsweise mindestens 5000, und ganz besonders mindestens 8000 beträgt.

13. Verfahren gemäß dem vorhergehenden Anspruch, dadurch gekennzeichnet, daß man die Reagentien unter solchen Bedingungen einführt, daß die Geschwindigkeit des gasförmigen Gemisches der eingeführten Reagentien zwischen 3 und 15 m/s für einen Rohrdurchmesser des Reaktionsgefäßes zwischen 2 und 6 mm beträgt.

14. Verfahren gemäß einem der Ansprüche 1 bis 11, dadurch gekennzeichnet, daß man die gegebenenfalls verdünnten Reagentien unter solchen Bedingungen einführt, daß man auf dem Niveau der Kontaktzone dieser Reagentien einen laminaren Betriebszustand hat.

15. Verfahren gemäß einem der vorhergehenden Ansprüche, dadurch gekennzeichnet, daß man dem Gemisch der Gase, erhalten nach dem Kontaktieren der Reagentien, ein Abfließen in der Nähe vom Typ Druckkolben verleiht.

16. Verfahren gemäß einem der vorhergehenden Ansprüche, dadurch gekennzeichnet, daß die Verweilzeit der Reaktionsteilnehmer in dem Reaktor zwischen 1 und 15 Sekunden, vorzugsweise zwischen 3 und 6 Sekunden beträgt.

17. Verfahren gemäß einem der vorhergehenden Ansprüche, dadurch gekennzeichnet, daß man das Inkontaktbringen der Reagentien bei einer Temperatur zwischen 250 und 500 °C, besonders bei einer Temperatur zwischen 300 und 400 °C bewirkt.

18. Verfahren gemäß einem der vorhergehenden Ansprüche, dadurch gekennzeichnet, daß man das Verfahren bei einem Druck nahe dem Atmosphärendruck durchführt.

19. Verfahren gemäß einem der vorhergehenden Ansprüche, dadurch gekennzeichnet, daß man die aromatische Verbindung, welche durch mindestens zwei Isocyanatgruppen substituiert ist, erhalten nach dem Verfahren gemäß einem der vorhergehenden Ansprüche, durch selektive Kondensation derselben in einem Lösungsmittel, dessen Siedetemperatur höher ist als die Zersetzungstemperatur des der vorerwähnten aromatischen Verbindung entsprechenden Carbamylchlorids, abtrennt.
